(19) Europäisches Patentamt
European Patent Office
Office européen des brevets

(11) Veröffentlichungsnummer: **0 011 790**
**B1**

(12) # EUROPÄISCHE PATENTSCHRIFT

(45) Veröffentlichungstag der Patentschrift: **15.07.81**

(51) Int. Cl.³: **C 07 C 85/145,** C 07 C 87/60

(21) Anmeldenummer: **79104558.6**

(22) Anmeldetag: **19.11.79**

(54) **Verfahren zur Herstellung von Nitrodiphenylamin-Derivaten.**

(30) Priorität: **29.11.78 DE 2851514**

(43) Veröffentlichungstag der Anmeldung: **11.06.80 Patentblatt 80/12**

(45) Bekanntmachung des Hinweises auf die Patenterteilung: **15.07.81 Patentblatt 81/28**

(84) Benannte Vertragsstaaten: **BE DE FR GB IT NL**

(56) Entgegenhaltungen: **DE-A-2 148 223**

(73) Patentinhaber: **BAYER AG, Zentralbereich Patente, Marken und Lizenzen, D-5090 Leverkusen 1, Bayerwerk (DE)**

(72) Erfinder: **Fauss, Rudolf, Dr., Gerstenkamp 10, D-5000 Köln 80 (DE)**
Erfinder: **Wagner, Kuno, Am Kiesberg 8, D-5090 Leverkusen (DE)**
Erfinder: **Mazanek, Jan, Dr., Jacob-Böhme-Strasse 13, D-5000 Köln 80 (DE)**

**EP 0 011 790 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung von Nitrodiphenylamin-Derivaten

Die Erfindung betrifft ein Verfahren zur Herstellung von Nitrodiphenylamin-Derivaten.

4-Nitrodiphenylamin kann durch Umsetzung von 4-Nitrochlorbenzol mit Anilin (DE-OS 10 30 834) oder durch Umsetzung von 4-Nitrochlorbenzol mit Formalinid (DE-OS 1056615) erhalten werden.

Weiterhin ist es bekannt (US-PS 3847990), 4-Nitrodiphenylamin durch Umsetzung von 4-Nitrophenol mit Phenylisocyanat in Gegenwart von alkalischen Katalysatoren und Nitrobenzol als Lösungsmittel bei Temperaturen um 200°C zu erhalten. Dieses Verfahren hat einen entscheidenden sicherheitstechnischen Nachteil: Aus dem eingesetzten 4-Nitrophenol und den als alkalische Katalysatoren besonders geeigneten Alkaliacetaten, -hydroxiden und -carbonaten können sich Alkali-4-nitrophenolate bilden, die aus der Nitrobenzol-Lösung, besonders gegen Ende der Reaktion, in fester Form ausfallen. Diese Alkali-4-nitrophenolate sind bekanntlich im festen Zustand bei höheren Temperaturen explosionsgefährlich.

Es wurde ein Verfahren zur Herstellung von Nitrodiphenylamin-Derivaten durch Reaktion eines Nitrophenols der Formel

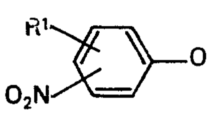

(I),

in der

$R^1$ für Wasserstoff, Alkyl, Cycloalkyl, Aryl, Alkoxy, Aryloxy, Acyl, Halogen, Nitro oder Cyano steht,

mit einem aromatischen Isocyanat der Formel

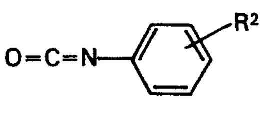

(II),

in der

$R^2$ für Wasserstoff, Alkyl, Cycloalkyl, Aryl, Alkoxy, Aryloxy, Acyl, Halogen, Nitro oder Cyano steht,

in Gegenwart von einer Base im Temperaturbereich von 100 bis 250°C, wobei das intermediär entstehende Urethan im Reaktionsgemisch decarboxyliert wird, gefunden, das dadurch gekennzeichnet ist, dass man in Tetramethylensulfon als Reaktionsmedium arbeitet.

Im erfindungsgemässen Verfahren werden bevorzugt Nitrophenole der Formel (I) eingesetzt, bei denen $R^1$ für Wasserstoff, Alkyl, Phenyl, Alkoxy, Phenyloxy oder Halogen steht.

Besonders bevorzugt ist im erfindungsgemässen Verfahren der Einsatz von Nitrophenolen der Formel (I), bei denen $R^1$ für Wasserstoff, Methyl, Methoxy, Phenyloxy oder Chlor steht.

Bevorzugt wird im erfindungsgemässen Verfahren ein aromatisches Isocyanat der Formel (II) eingesetzt, bei dem $R^2$ für Wasserstoff, Alkyl, Phenyl, Alkoxy, Phenyloxy oder Halogen steht.

Besonders bevorzugt ist im erfindungsgemässen Verfahren der Einsatz eines aromatischen Isocyanats der Formel (II), bei dem $R^2$ für Wasserstoff, Methyl, Methoxy, Phenyloxy oder Chlor steht.

Als Alkyl seien geradkettige oder verzweigte Kohlenwasserstoffreste mit 1 bis 6 Kohlenstoffatomen genannt, beispielsweise Methyl, Äthyl, Propyl, Isopropyl, Butyl, Isobutyl, Pentyl, Isopentyl, Hexyl oder Isohexyl. Bevorzugtes Alkyl sind Kohlenwasserstoffreste mit 1 bis 4 Kohlenstoffatomen, beispielsweise Methyl, Äthyl, Propyl, Isopropyl, Butyl oder Isobutyl. Ganz bevorzugtes Alkyl ist der Methylrest.

Als Cycloalkyl seien ringförmige Kohlenwasserstoffreste mit 5 bis 7 Kohlenstoffatomen, die gegebenenfalls durch niederes Alkyl substituiert sein können, genannt, beispielsweise Cyclopentyl, Methyl-cyclopentyl, Cyclohexyl, Methyl-cyclohexyl, Cycloheptyl oder Methyl-cyclo-heptyl.

Als Aryl seien aromatische Kohlenwasserstoffreste mit 6 bis 14 Kohlenstoffatomen genannt, beispielsweise Phenyl, Naphthyl oder Anthryl. Bevorzugtes Aryl ist der Phenylrest.

Als Alkoxy seien Reste genannt, die sich von einem niederen Alkohol mit 1 bis 4 Kohlenstoffatomen ableiten, beispielsweise Methoxy, Äthoxy, Propoxy, Isopropoxy, Butoxy oder Isobutyloxy. Bevorzugtes Alkoxy ist der Methoxyrest.

Als Aryloxy seien Reste genannt, die sich von einer aromatischen Hydroxylverbindung ableiten, beispielsweise Phenoxy, Naphthyloxy oder Anthryloxy. Bevorzugtes Aryloxy ist der Phenoxyrest.

Als Acyl seien Alkylcarbonylreste mit 2 bis 5 Kohlenstoffatomen genannt, beispielsweise Acetyl, Propionyl, Butyryl oder Valeroyl. Bevorzugtes Acyl ist der Acetylrest.

Als Halogen seien Fluor, Chlor oder Brom, bevorzugt Chlor, genannt.

Als erfindungsgemäss einzusetzende Nitrophenole seien beispielsweise genannt:
p-Nitrophenol, o-Nitrophenol, 2,4-Dinitrophenol, 4-Nitro-2-chlorphenol, 4-Nitro-3-chlorphenol, 3-Chlor-2-nitrophenol, 4-Chlor-2-nitrophenol, 5-Chlor-2-nitrophenol, 6-Chlor-2-nitrophenol, 2,6-Dichlor-4-nitrophenol, 4-Nitro-orthokresol, 4-Nitro-metakresol, 6-Nitro-orthokresol, 6-Nitro-metakresol, 4-Nitro-2-isopropylphenol, 4-Nitro-2-isobutylphenol, 4-Nitro-2-cyclohexylphenol.

Als erfindungsgemäss einsetzbare aromatische Isocyanate seien beispielsweise genannt:
Phenylisocyanat, m-Methylphenylisocyanat, p-Methylphenylisocyanat, m-Isobutylphenylisocyanat, m-2-Äthylhexylphenylisocyanat, m-Cyclohexylphenylisocyanat, m-Chlorphenylisocyanat, p-Chlorphenylisocyanat, m-Nitrophenylisocyanat, p-Nitrophenylisocyanat.

Selbstverständlich kann anstelle eines gegebenenfalls substituierten Nitrophenols und eines gegebenenfalls substituierten aromatischen Iso-

cyanats, die im Reaktionsgemisch unter Bildung eines Urethans reagieren, auch ein Urethan der Formel

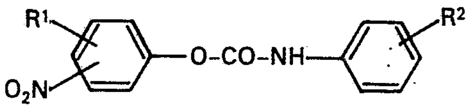

(III),

in der

$R^1$ und $R^2$ die obgenannte Bedeutung besitzen, eingesetzt werden, welches sodann in Gegenwart einer Base auf eine Temperatur von etwa 100–250 °C in Tetramethylensulfon als Reaktionsmedium erhitzt wird.

Urethane der Formel (III) sind nach bekannten Verfahren herstellbar, beispielsweise durch Reaktion gegebenenfalls substituierter Nitrophenole mit Isocyanaten (Ind. Eng. Chem., Anal. Ed. 16, 304 (1944)) oder durch Reaktion von gegebenenfalls substituierten Nitrophenyl-Chlorkohlensäureestern mit gegebenenfalls substituierten Aminen (Liebigs Ann. Chem. 562, 205 (1949)).

Die Ausgangsprodukte im erfindungsgemässen Verfahren können im Verhältnis von 0,5 bis 5 Mol aromatisches Isocyanat zu 1 Mol Nitrophenol, bevorzugt im Verhältnis von 1 bis 2 Mol aromatisches Isocyanat zu 1 Mol Nitrophenol, eingesetzt werden.

Als Basen für das erfindunsgemässe Verfahren seien basische anorganische oder organische Verbindungen genannt.

Als basische anorganische Verbindungen seien basisch reagierende Verbindungen der Alkali- und Erdalkalimetalle genannt, beispielsweise deren Hydroxide, Carbonate oder basisch reagierende Salze organischer Säuren, wie die Acetate, Formiate, Benzoate oder Phthalate.

Als basische organische Verbindungen seien beispielsweise Phenolate oder substituierte Phenolate, wie Nitrophenolate, von Alkalimetallen, genannt. Weiterhin seien als basische organische Verbindungen tertiäre hochsiedende Amine und Phosphine genannt. Beispiele für die letzteren sind N-Methyl-bis-(β-hydroxyäthylphenyläther)-amin und Tri-n-butyl-phosphin.

Bevorzugte Basen für das erfindungsgemässe Verfahren sind die Carbonate oder Hydroxide von Natrium oder Kalium.

Die Base kann in Mengen von 0,5 bis 50 Mol-%, bevorzugt in Mengen von 1 bis 10 Mol-%, bezogen auf das eingesetzte Nitrophenol bzw. Nitrophenyl-N-phenylurethan, eingesetzt werden.

Als Reaktionstemperatur für das erfindungsgemässe Verfahren sei der Bereich von 100 bis 250 °C, bevorzugt der Bereich von 150 bis 220 °C, genannt.

Die Nitrodiphenylamin-Derivate, die im erfindungsgemässen Verfahren hergestellt werden können, können durch die Formel

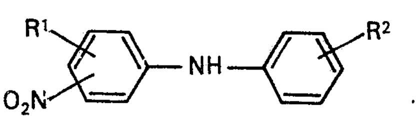

(IV)

in der

$R^1$ und $R^2$ gleich oder verschieden sein können und für Wasserstoff, Alkyl, Cycloalkyl, Aryl, Alkoxy, Aryloxy, Acyl, Halogen, Nitro oder Cyano stehen, dargestellt werden.

Als Beispiele für Verbindungen der Formel (IV) seien genannt:

2-Nitrodiphenylamin, 3-Nitrodiphenylamin, 4-Nitrodiphenylamin, 2-Nitro-4-methyl-diphenylamin, 4-Nitro-2-methyl-diphenylamin, 4-Nitro-2-phenyl-diphenylamin, 4-Nitro-2-cyclohexyl-diphenylamin, 4-Nitro-2-methoxy-diphenylamin, 2-Nitro-4-acetyl-diphenylamin, 2-Nitro-4-acetyldiphenylamin, 2-Nitro-4-chlor-diphenylamin, 4-Nitro-2-cyano-diphenylamin, 2,4-Dinitro-diphenylamin, 2-Nitro-4′-methyl-diphenylamin, 4-Nitro-4′-phenyl-diphenylamin, 4-Nitro-2′-methoxy-diphenylamin, 2-Nitro-4′-acetyl-diphenylamin, 4-Nitro-3′-chlor-diphenylamin, 4-Nitro-3′-methyl-diphenylamin, 2,4′-Dinitro-diphenylamin, 2-Nitro-2′,4-dimethyl-diphenylamin, 4-Nitro-2,4′-dichlor-diphenylamin, 3-Nitro-2,4′-dimethyl-diphenylamin.

Das erfindungsgemässe Verfahren kann wie folgt durchgeführt werden:

Das gegebenenfalls substituierte Nitrophenol wird in Tetramethylensulfon (Sulfolan) gelöst und mit der basischen Verbindung versetzt. In dem Fall, dass die basische Verbindung in Form einer wässrigen Lösung zugesetzt wird, wird anschliessend dieses Wasser durch Destillation entfernt. Anschliessend wird das Gemisch auf die gewünschte Temperatur erhitzt und das gegebenenfalls substituierte aromatische Isocyanat unter kräftigem Rühren zugetropft.

Es setzt eine Kohlendioxid-Entwicklung ein, die nach einer halben bis 4 Stunden, je nach Ansatzgrösse, beendet sein kann. Anschliessend wird die basische Verbindung durch Zugabe von Mineralsäure neutralisiert. Nach dem Abfiltrieren von ausgefallenen Salzen wird das Sulfolan durch Destillation weitgehend entfernt. Der feste Rückstand der noch 1 bis 5 Gew.-% Sulfolan enthalten kann, wird durch übliche Aufarbeitungsmethoden, beispielsweise durch Umkristallisation aus Xylol, weiter gereinigt. Es ist möglich, die Mutterlauge des Umkristallisationsschrittes vom Lösungsmittel zu befreien und den dabei erhaltenen Rückstand in einem neuen Ansatz des erfindungsgemässen Verfahrens mit einzusetzen.

Geht man in der erfindungsgemässen Variante des Verfahrens von einem gegebenenfalls substituierten Nitrophenyl-N-phenylurethan der Formel (III) aus, so kann dieses im Sulfolan gelöst und mit der alkalischen Verbindung versetzt werden. Daraufhin wird dieses Reaktionsgemisch auf die gewünschte Reaktionstemperatur erhitzt und weiter wie oben verfahren.

Die so erhaltenen gereinigten Produkte besitzen eine Reinheit von mehr als 96%. Das erfindungsgemässe Verfahren weist folgende Vorteile auf:

1. Aus dem Reaktionsansatz fällt kein festes Alkali-4-nitrophenolat aus. Dadurch ist das erfindungsgemässe Verfahren ungefährlich im Ge-

gensatz zum Verfahren US-PS 3 847 990.
2. Das als Nebenprodukt in geringen Mengen zu erwartende Triphenylisocyanurat, das sich aus dem aromatischen Isocyanat bilden kann, bleibt, im Gegensatz zum Nitrophenol-Verfahren, in Lösung, so dass Verkrustungen der Reaktionsapparatur vermieden werden.
3. Die Ausbeute an aufgearbeitetem, reinem Nitrodiphenylamin liegt im erfindungsgemässen Verfahren um bis zu 10% höher als im Verfahren der genannten US-PS.
4. Die Reaktionsgeschwindigkeit ist unter sonst gleichen Bedingungen im erfindungsgemäss verwendeten Sulfolan etwa 3mal grösser als im Nitrobenzol. Gleichzeitig kann in Sulfolan mit höheren Konzentrationen an Reaktionskomponenten, verglichen mit Nitrobenzol, gearbeitet werden. Dadurch ergibt sich eine beträchtliche Steigerung der Raum/Zeit-Ausbeute.

Es ist überraschend, dass durch die erfindungsgemässe Anwendung von Tetramethylensulfon als Reaktionsmedium ein sicherheitstechnisch befriedigendes Verfahren erhalten werden konnte, das zudem eine höhere Ausbeute liefert als das Verfahren des Standes der Technik. Dies ist um so überraschender, als der hohe Siedepunkt des Tetramethylensulfons und seine hohe Polarität eine schwierige Trennung vom Reaktionsprodukt erwarten liessen.

Nitrodiphenylamin und seine Derivate sind wichtige Zwischenprodukte für die Synthese von p-Alkylamino- und p-Dialkylamino-diphenylamin, die als Stabilisatoren die Oxidation von natürlichen und synthetischen Kautschuken durch Sauerstoff weitgehend verhindern. (US-PS 3 163 616)

Beispiel 1
150,2 g (1,08 Mol) p-Nitrophenol werden in 750 ml Sulfolan gelöst und mit 5,52 g (0,04 Mol) Kaliumcarbonat in 10 ml Wasser versetzt. Im Vakuum wird das Wasser abdestilliert und bei 205 °C werden 129,7 g (1,09 Mol) Phenylisocyanat während 1 Stunde unter kräftigem Rühren zugetropft. Nach weiteren 1,5 Stunden haben sich ca. 24 l $CO_2$ entwickelt. Bei 150 °C wird mit 2,2 ml $H_2SO_4$ conc. versetzt, kurz nachgerührt und über eine Glassinternutsche abgesaugt. Das Sulfolan wird im Dünnschichtverdampfer bei 1 bis 2 Torr (133,3–266,6 Pa) weitgehend abdestilliert.

Es bleiben 234 g Rohprodukt, das aus 620 ml Xylol umkristallisiert wird. Es werden 185 g (86,5% der theoretischen Ausbeute) 4-Nitrodiphenylamin erhalten. Fp.: 129 bis 130 °C.

Beispiel 2
Es wird analog Beispiel 1 vorgegangen, jedoch bei Verwendung von
270,4 g (1,94 Mol) p-Nitrophenol,
750 ml Sulfolan, 9,94 g (0,072 Mol) Kaliumcarbonat,
214 g (1,89 Mol) Phenylisocyanat,
4 ml Schwefelsäure conc.
Es wurden 182,8 g = 85,4% der theoretischen Ausbeute an 4-Nitrodiphenylamin erhalten; Fp.: 130 bis 131 °C.

Beispiel 3
Wie Beispiel 2, jedoch mit 8,1 g (0,14 Mol) Kaliumhydroxid. Es konnten 188,1 g = 87,9% der theoretischen Ausbeute an 4-Nitrodiphenylamin isoliert werden; Fp.: 129 bis 130 °C.

Beispiel 4
Es wird vorgegangen, wie in Beispiel 1 beschrieben worden ist, jedoch wird mit 167,4 g (1,09 Mol) m-Chlorphenylisocyanat gearbeitet. Es werden 165,3 g (66,5% der theoretischen Ausbeute) an 4-Nitro-3′-chlordiphenylamin erhalten. Fp.: 127 bis 128 °C.

Beispiel 5
Es wird vorgegangen, wie in Beispiel 1 beschrieben worden ist, jedoch wird mit 167,4 g (1,09 Mol) p-Chlorphenylisocyanat gearbeitet. Es werden 159 g (64% der theoretischen Ausbeute) an 4-Nitro-4′-chlordiphenylamin erhalten. Fp.: 179 bis 180 °C.

Beispiel 6
Es wird vorgegangen, wie in Beispiel 1 beschrieben worden ist, jedoch wird mit 145,1 g (1,09 Mol) m-Methylisocyanat gearbeitet. Es werden 182,6 g (80,1% der theoretischen Ausbeute) an 4-Nitro-3′-methyldiphenylamin erhalten. Fp.: 129 bis 130 °C.

**Patentansprüche**

1. Verfahren zur Herstellung von Nitrodiphenylamin-Derivaten durch Reaktion eines Nitrophenols der Formel

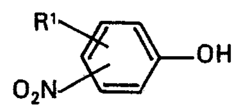

in der
$R^1$ für Wasserstoff, Alkyl, Cycloalkyl, Aryl, Alkoxy, Aryloxy, Acyl, Halogen, Nitro oder Cyano steht,
mit einem aromatischen Isocyanat der Formel

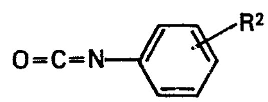

in der
$R^2$ für Wasserstoff, Alkyl, Cycloalkyl, Aryl, Alkoxy, Aryloxy, Acyl, Halogen, Nitro oder Cyano steht,
in Gegenwart einer Base im Temperaturbereich von 100 bis 250 °C, wobei das intermediär entstehende Urethan im Reaktionsgemisch decarboxyliert wird, dadurch gekennzeichnet, dass man in Tetramethylensulfon als Reaktionsmedium arbeitet.

2. Verfahren nach Anspruch 1, dadurch gekennzeichnet, dass anstelle des gegebenenfalls

substituierten Nitrophenols und des gegebenenfalls substituierten aromatischen Isocyanats, die im Reaktionsgemisch unter Bildung eines Urethan der Formel

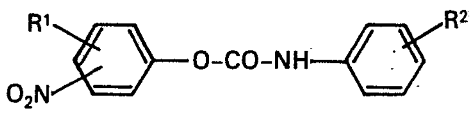

reagieren, wobei $R^1$ und $R^2$ die in Anspruch 1 genannte Bedeutung haben, dieses Urethan als Ausgangsverbindung eingesetzt wird.

**Claims**

1. A process for the preparation of nitro-diphenyl amine derivatives by reacting a nitrophenol of the formula

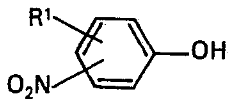

in which
$R^1$ denotes hydrogen, alkyl, cycloalkyl, aryl, alkoxy, aryloxy, acyl, halogen, nitro or cyano,
with an aromatic isocyanate of the formula

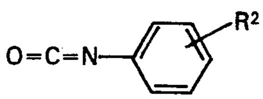

in which
$R^2$ denotes hydrogen, alkyl, cycloalkyl, aryl, alkoxy, aryloxy, acyl, halogen, nitro or cyano,
in the presence of a base within a temperature range of 100° to 250°C, the urethane forming intermediately being decarboxylated in the reaction mixture, characterised in that the process is conducted in tetramethylene sulphone as the reaction medium.

2. A process according to claim 1, characterised in that instead of the optionally substituted nitrophenol and the optionally substituted aromatic isocyanate, which react in the reaction mixture accompanied by the formation of a urethane of the formula

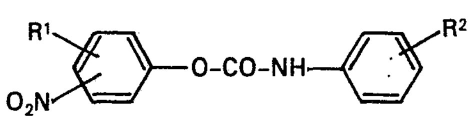

in which $R^1$ and $R^2$ have the meaning mentioned in claim 1, this urethane is used as starting compound.

**Revendications**

1. Procécé de fabrication de dérivés de nitrodiphénylamine par réaction d'un nitrophénol de formule:

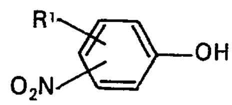

dans laquelle
$R^1$ représente de l'hydrogène, alcoyle, cycloalcoyle, aryle, alcoxy, aryloxy, acyle, halogène, nitro ou cyano,
avec un isocyanate aromatique de formule:

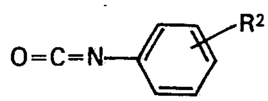

dans laquelle
$R^2$ représente de l'hydrogène, alcoyle, cycloalcoyle, aryle, alcoxy, aryloxy, acyle, halogène, nitro ou cyano,
en présence d'une base dans l'intervalle de température de 100 à 250 °C, l'uréthane intermédiairement obtenu dans le mélange de réaction étant décarboxylé, caractérisé en ce qu'on opère dans de la tétraméthylène sulfone comme milieu de réaction.

2. Procédé selon la revendication 1, caractérisé en ce qu'au lieu du nitrophénol éventuellement substitué et de l'isocyanate aromatique éventuellement substitué qui réagissent dans le milieu de réaction avec formation d'un uréthane de formule:

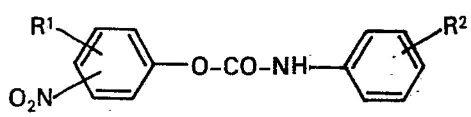

$R^1$ et $R^2$ ayant la signification indiquée à la revendication 1, on utilise cet uréthane comme composé de départ.